# EUROPÄISCHE PATENTSCHRIFT

(11) **EP 0 559 116 B1**
(45) Veröffentlichungstag und Bekanntmachung des Hinweises auf die Patenterteilung: **28.05.1997**
(21) Anmeldenummer: 93103221.3
(22) Anmeldetag: 01.03.1993
(51) Int. Cl.: C12P 17/12, C07D 213/80, C07D 241/24, C12N 1/20

(54) **Mikrobiologisches Verfahren zur Hydroxylierung von Stickstoff-Heterocyclus-Carbonsäuren**
Microbiological process for the hydroxylation of nitrogen containing heterocyclic carbonic acids
Procédé microbiologique pour l'hydroxylation d'acide carbonique hétérocyclique contenant de l'azote

(30) Priorität: 04.03.1992 CH 672/92
(43) Veröffentlichungstag der Anmeldung: 08.09.1993
(73) Patentinhaber: LONZA AG, CH-3945 Gampel/Wallis (CH)
(72) Erfinder: Kiener, Andreas, Dr., Visp (Kanton Wallis) (CH); Tschech, Andreas, Dr., Aarau (Kanton Aargau) (CH); Tinschert, Andreas, Brig (Kanton Wallis) (CH); Heinzmann, Klaus, Visperterminen (Kanton Wallis) (CH)
(74) Vertreter: Weinhold, Peter, Dr.

(56) Entgegenhaltungen:
- EP-A- 0 434 035
- CHEMICAL ABSTRACTS, vol. 66, no. 21, 22. Mai 1967, Columbus, Ohio, US; abstract no. 94996s, HENRYK FOKS ET AL. '2-Pyrazinecarboxylic acid N-oxides. II. Reactions of the derivatives of 2-pyrazinecarboxylic acid N-oxide with acetic anhydride.' Seite 8898 ;
- CHEMICAL ABSTRACTS, vol. 107, no. 25, 21. Dezember 1987, Columbus, Ohio, US; abstract no. 228395w, YAMAMOTO, TETSUYA ET AL. 'In vitro conversion of pyrazinamide into 5-hydroxypyrazinamide and that of pyrazinoic acid into 5-hydroxypyrazinoic acid by xanthine oxidase from human liver.' Seite 15 ;
- CHEMICAL ABSTRACTS, vol. 101, no. 1, 2. Juli 1984, Columbus, Ohio, US; abstract no. 7119h, LADUREE,DANIEL ET AL 'Synthesis of 9H-pyrrolo (1,2-a)-1,4-diazaindol-9-0ne.' Seite 609 ;
- CHEMICAL ABSTRACTS, vol. 103, no. 19, 11. November 1985, Columbus, Ohio, US; abstract no. 160400d, Seite 700 ;

## Beschreibung

Die Erfindung betrifft ein neues mikrobiologisches Verfahren zur Herstellung von Hydroxy-Stickstoff-Heterocyclus-Carbonsäuren oder deren lösliche Salze der allgemeinen Formel 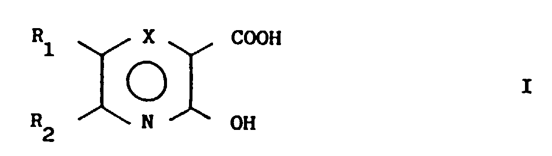 ausgehend von den entsprechenden Stickstoff-Heterocyclus-Carbonsäuren, sowie neue für das Verfahren geeignete Mikroorganismen.

Im folgenden werden als Stickstoff-Heterocyclus-Carbonsäuren, hydroxyliert oder nicht hydroxyliert, auch deren lösliche Salze, wie beispielsweise deren Alkali- oder Ammoniumsalze verstanden.

2-Hydroxy-Stickstoff-Heterocyclus-Carbonsäuren sind wichtige Zwischenprodukte in Wirkstoffsynthesen. Beispielsweise dient die 2-Hydroxynikotinsäure als Ausgangsmaterial zur Herstellung von 2-Chlornikotinsäure (US-PS 4 081 451), welches beispielsweise ein wichtiges Zwischenprodukt zur Herstellung von Pharmazeutika darstellt. (Ann. Pharm. Fr., 1980, 38(3), S. 243 - 252).

Bisher sind keine allgemeinen mikrobiologischen Verfahren zur Herstellung von Hydroxy-Stickstoff-Heterocyclus-Carbonsäuren bekannt.

EP-A-0 434 035 offenbart ein Verfahren zur Herstellung von 6-Hydroxynikotinsäure aus Nikotinsäure durch enzymatische Hydroxylierung in Gegenwart von Mikroorganismen der Gattungen Pseudomonas, Bacillus oder Achromobacter. Durch Einhalten eines bestimmten Konzentrationsbereichs während der Nikotinsäurezugabe kann die Biomassebildung im selben Verfahrensschritt wie die Produktbildung erfolgen, ohne daß es zu Produktverlusten durch weiteren Abbau kommt.

Aufgabe der Erfindung war, ein einfaches und ökologisches mikrobiologisches Verfahren zur Herstellung von chemisch schwer zugänglichen 2-Hydroxy-Stickstoff-Heterocyclus-Carbonsäuren zur Verfügung zu stellen.

Diese Aufgabe wurde mit dem erfindungsgemässen Verfahren gemäss Patentanspruch 1 und mit dem neuen Mikroorganismen gemäss Patentanspruch 6 gelöst.

Erfindungsgemäss wird das Verfahren derart ausgeführt, dass man eine Stickstoff-Heterocyclus-Carbonsäure der allgemeinen Formel 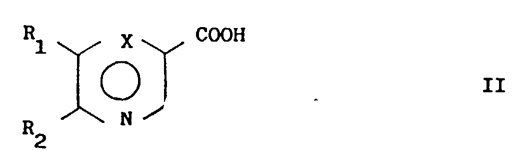 worin R₁ und R₂ gleich oder verschieden sind und ein Wasserstoffatom, ein Halogenatom oder eine C₁-C₄-Alkylgruppe und X ein Stickstoffatom oder eine CR₃-Funktion bedeuten, worin R₃ ein Wasserstoff- oder ein Halogenatom bedeutet, als Substrat, mittels 6-Methylnikotinsäure-verwertenden Mikroorganismen, enthaltend eine spezifische Hydroxylase, in die Hydroxy-Stickstoff-Heterocyclus-Carbonsäure der allgemeinen Formel 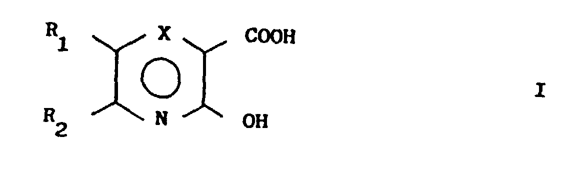 worin R₁, R₂ und X die genannte Bedeutung haben, überführt.

Unter dem Begriff "6-Methylnikotinsäure-verwertende Mikroorganismen enthaltend eine spezifische Hydroxylase" ist folgendes zu verstehen:
Züchtet man beispielsweise aus Klärschlamm als Inokulum mit 6-Methylnikotinsäure Biomasse an, erhält man 6-Methylnikotinsäure-verwertende Mikroorganismen, dh. Mikroorganismen, die mit 6-Methylnikotinsäure als einzige Kohlenstoff-, Stickstoff- und Energiequelle wachsen. Selektioniert man nun nach fachmännisch üblichen Methoden die Mikroorganismen, die 6-Methylnikotinsäure über 2-Hydroxy-6-methylnikotinsäure als zwischenprodukt vollständig abbauen, erhält man Mikroorganismen, die eine spezifische Hydroxylase enthalten, d.h. Mikroorganismen, die spezifisch das zwischen der Säurefunktion und dem Stickstoffatom liegende einzige Kohlenstoffatom hydroxylieren.

Prinzipiell sind für das Verfahren alle Mikroorganismen geeignet, die nach diesem Prinzip, beispielsweise aus Klärschlamm oder Erdproben, selektioniert sind. Diese Mikroorganismen sind in der Literatur nicht beschrieben und Bestandteil der Erfindung.

Zweckmässig werden die Mikroorganismen mit der Bezeichnung Ki101 (DSM 6920) sowie deren Deszendenten und Mutanten für das Verfahren eingesetzt. Diese sind am 10.2.1992 bei der Deutschen Sammlung für Mikroorganismen und Zellkulturen GmbH, Mascheroderweg 1b, D-3300 Braunschweig, hinterlegt worden.

Wissenschaftliche Beschreibung von Ki 101 (DSM 6920):

| | |
|---|---|
| Zellform | winzige Stäbchen |
| Breite µm | 0.4-0.5 |
| Länge µm | 1.0-1.5 |
| Beweglichkeit | - |
| Geisseln | - |
| Gram-Reaktion | - |
| Lyse durch 3% KOH | + |
| Aminopeptidase (Cerny) | + |
| Sporen | - |
| Oxidase | + |
| Catalase | + |

Eine Gattung über 16S rRNA-Sequenzanalyse konnte nicht bestimmt werden.

Die Selektion und die Anzucht dieser Mikroorganismen, mit 6-Methylnikotinsäure, erfolgt an sich nach fachmännisch üblichen Methoden.

Zweckmässig erfolgt während der Selektion das "Screening" nach den Mikroorganismen unter aeroben Bedingungen. Vorzugsweise werden die Mikroorganismen während des "Screenings" ruhend (nicht unter Schütteln) kultiviert.

Die Menge an 6-Methylnikotinsäure beträgt zweckmässig während der Selektion und der Anzucht bis zu 1 Gew.%, vorzugsweise bis zu 0,5 Gew.%.

Selektion und Anzucht werden zweckmässig bei einem pH-Wert von 5 bis 9, vorzugsweise von 6 bis 8 durchgeführt.

Die Temperatur liegt zweckmässig während der Selektion und der Anzucht zwischen 15 und 50°C, vorzugsweise zwischen 25 und 40°C.

Üblicherweise erfolgt die Selektion und Anzucht in einem Mineralsalzmedium, vorzugsweise in dem Mineralsalzmedium dessen Zusammensetzung in Tabelle 1 beschrieben ist.

Ist eine zweckmässige optische Dichte bei 650 nm (OD₆₅₀) von 0,5 bis 100 erreicht, können die Mikroorganismen entweder nach fachmännisch üblichen Methoden geerntet werden oder das Substrat, die Stickstoff-Heterocyclus-Carbonsäure, kann direkt, für die eigentliche Umsetzung (Biotransformation) zu den Mikroorganismen hinzugegeben werden.
Die eigentliche Biotransformation erfolgt dann auf fachmännisch übliche Weise mit nicht wachsenden Zellen.

Als Substrate der allgemeinen Formel II können beispielsweise Nikotinsäure, Pyrazincarbonsäure oder deren halogenierte oder C₁-C₄-alkylierte Derivate dienen.

Als halogenierte Nikotinsäure- oder Pyrazincarbonsäure-Derivate werden vorzugsweise 6-Chlornikotinsäure, 5,6-Dichlornikotinsäure und 5-Chlorpyrazincarbonsäure hydroxyliert.

Als C₁-C₄-Pyrazincarbonsäure-Derivate wird vorzugsweise 5-Methylpyrazin-carbonsäure hydroxyliert.

Das Substrat kann für die Biotransformation kontinuierlich oder einmalig hinzugegeben werden. Zweckmässig erfolgt die Substratzugabe so, dass die Substratkonzentration im Kulturmedium 10 Gew.%, vorzugsweise 7 Gew.%, nicht übersteigt.

Als Medium können für die Biotransformation die fachmännisch üblichen angewendet werden. Vorzugsweise wird die Biotransformation in einem niedermolaren Phosphat-Puffer durchgeführt.

Üblicherweise wird die Biotransformation mit einer Mikroorganismen-Suspension durchgeführt, die eine optische Dichte bei 650 nm (OD₆₅₀) von 0,5 bis 100 aufweist, vorzugsweise von 5 bis 50.

Die Biotransformation wird zweckmässig bei einer Temperatur von 15 bis 50°C, vorzugsweise von 25 bis 35°C und bei einem pH von 5 bis 9, vorzugsweise von pH 6,5 bis 7,5, durchgeführt.

Nach einer üblichen Umsetzungsdauer von 5 h bis zu 3 Tagen, kann dann das hydroxylierte Produkt gemäss Formel I nach fachmännisch üblichen Methoden, beispielsweise durch Ansäuern des zellfreien Überstandes, isoliert werden.

Die nach diesem Verfahren hergestellten Hydroxy-Stickstoff-Heterocyclus-Carbonsäuren der allgemeinen Formel 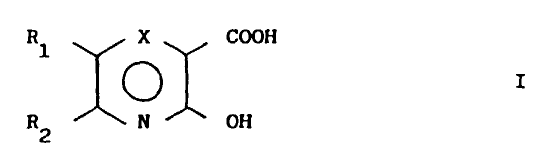 worin, wenn X ein Stickstoffatom bedeutet, R₁ und R₂ gleich oder verschieden sind und ein Wasserstoffatom, ein Halogenatom oder eine C₁-C₄-Alkylgruppe bedeuten, mit der Ausnahme, dass R₁ und R₂ nicht gemeinsam Wasserstoff bedeuten, sowie wenn X eine CH-Funktion bedeutet R₁ und R₂ ein Halogenatom bedeuten, sind in der Literatur nicht beschrieben.

Die bevorzugten Vertreter dieser neuen Verbindungen sind 5,6-Dichlor-2-hydroxynikotinsäure, 3-Hydroxy-5-methylpyrazincarbonsäure und 3-Hydroxy-5-chlorpyrazincarbonsäure.

### Beispiel 1:

### Isolierung von 6-Methyl-nikotinsäure-verwertenden Mikroorganismen

300 ml Erlenmeyerkolben wurden mit je 100 ml Mineralsalzmedium enthaltend 1 mmol 6-Methylnikotinsäure (Tabelle 1) gefüllt und mit 10 ml Klärschlamm aus der Kläranlage der Fa. LONZA AG in Visp, Schweiz oder mit Erdproben aus dem LONZA-Werk, Visp versetzt und ruhend bei 30°C inkubiert. Nach 10 Tagen ließ sich bei Wellenlängen zwischen 200 und 400 nm mittels eines UV-Spektrometers in einigen Ansätzen 2-Hydroxy-6-methylnikotinsäure spektrophotometrisch nachweisen. Diese Kulturen wurden anschliessend mehrere Male in frisches Mineralsalzmedium überimpft. Danach wurden die 2-Hydroxy-6-methylnikotinsäure-bildenden Zellsuspensionen auf dem gleichen Medium, enthaltend 1,6% (w/v) Agar ausgestrichen. Die Agarplatten wurden anschliessend bei 30°C in einer Atmosphäre enthaltend 4% O₂ und 96% N₂ inkubiert. Einzelkolonien wurden darauf in flüssiges Medium transferiert und ruhend inkubiert. 2-Hydroxy-6-methylnikotinsäure bildende Kolonien, die auch ein verlangsamtes Wachtstum zeigen, wurden wie oben spektrophotometrisch identifiziert. Der Bakterienstamm mit der Bezeichnung Ki101 (DSM 6920) bildete während des Wachstums auf 6-Methylnikotinsäure 2-Hydroxy-6-methylnikotinsäure.

### Beispiel 2:

### Mikrobielle Oxidation von Nikotinsäure zu 2-Hydroxynikotinsäure

Die Mikroorganismen mit der Bezeichnung Ki101 (DSM 6920) wurden in 800 ml Mineralsalzmedium (Tabelle 1) unter Zusatz von 8 mmol 6-Methylnikotinsäure in einem 1 l Fernbach-Kolben bei 30°C auf einem Schüttler bei 100 UpM inkubiert. Das Inokulum betrug 5% (v/v). Nach 5 Tagen betrug die OD₆₅₀ ca. 0,5. Danach wurden die Zellen geerntet und einmal mit 50 mM Phosphatpuffer, pH 7,0 gewaschen. Anschliessend wurden die Zellen in 20 ml 50 mM Phosphatpuffer, pH 7,0, enthaltend 200 mg (1,38 mmol) Nikotinsäure-Natriumsalz resuspendiert. Die OD₆₅₀ betrug 20. Nach 6 h Inkubation auf einem Schüttler bei 30°C, konnte mittels Dünnschichtchromatographie keine Nikotinsäure mehr nachgewiesen werden. Danach wurde die Biomasse abzentrifugiert und der Überstand auf pH 2,5 angesäuert, um die 2-Hydroxynikotinsäure auszufällen.
Mittels ¹H-NMR (DMSO) Analyse konnte keine Verunreinigung im isolierten Produkt nachgewiesen werden. Insgesamt konnten 140 mg (1 mmol) 2-Hydroxynikotinsäure entsprechend einer Ausbeute von 72% bez. Nikotinsäure isoliert werden.

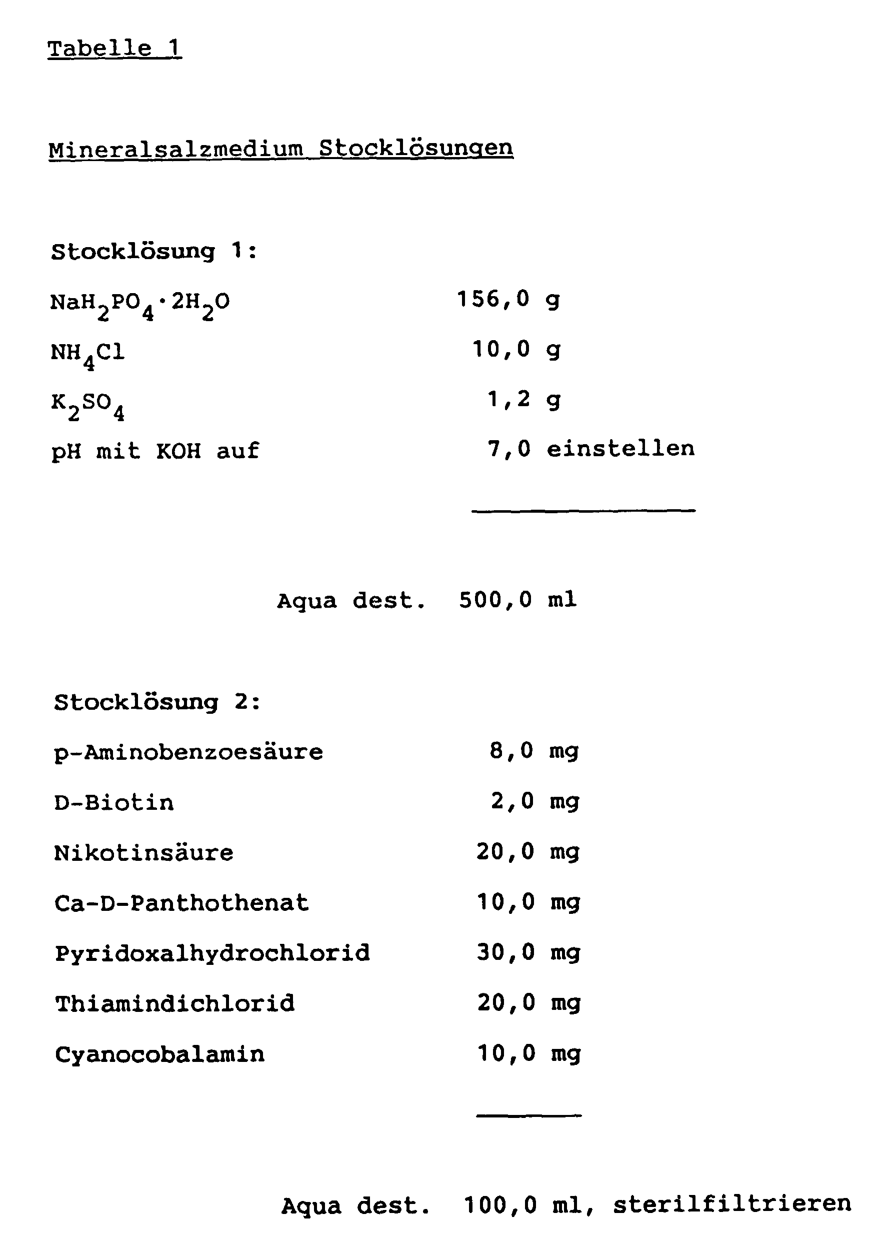

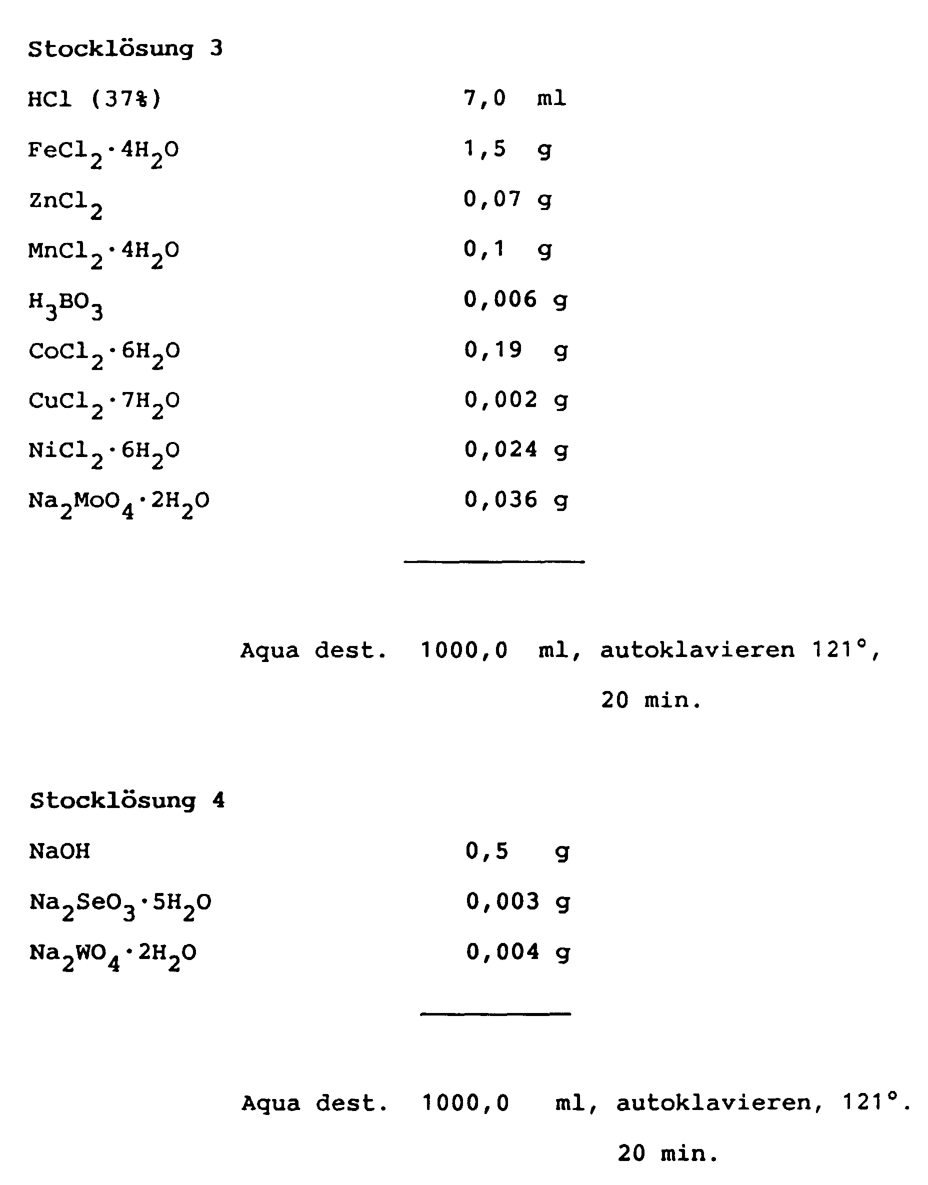

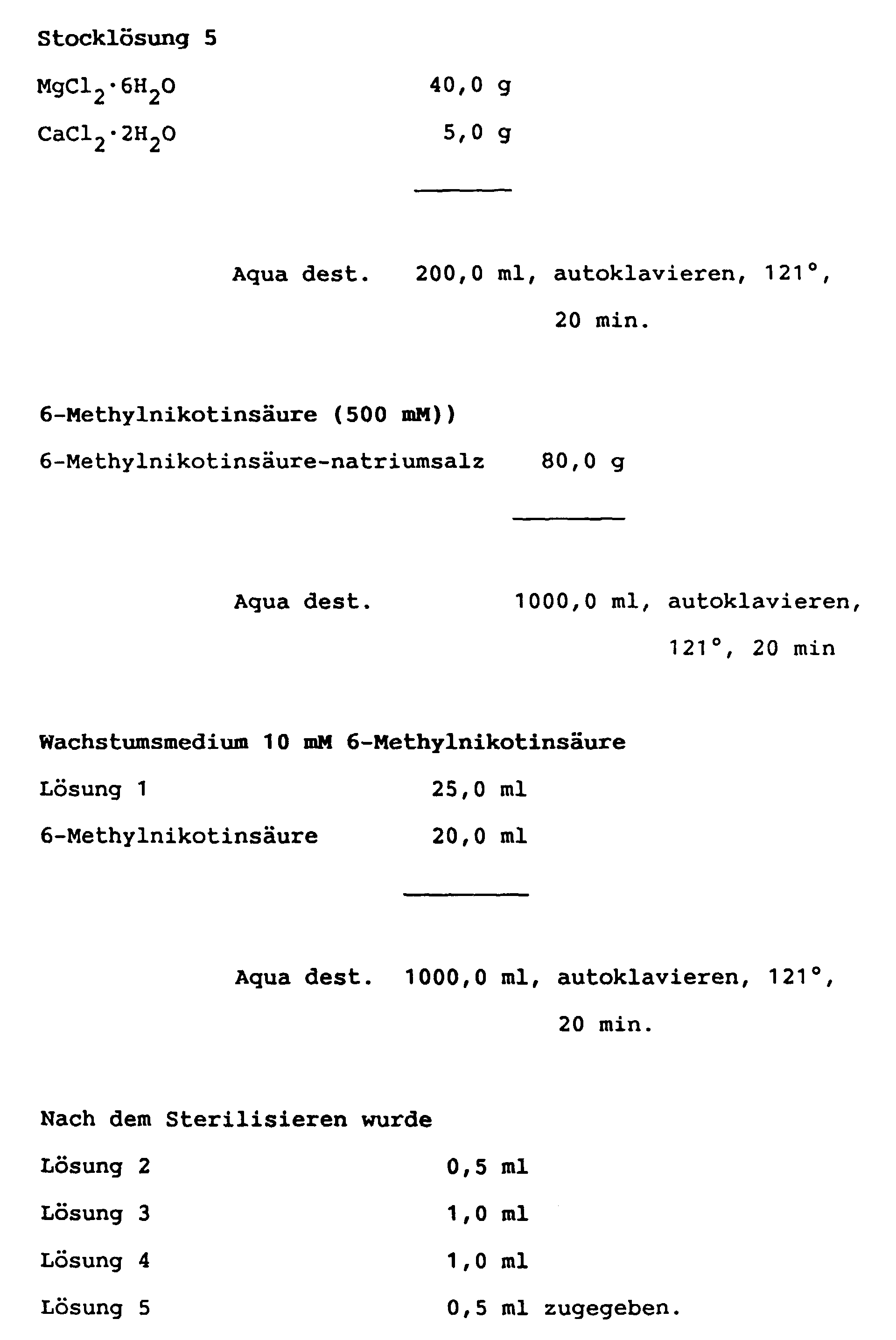

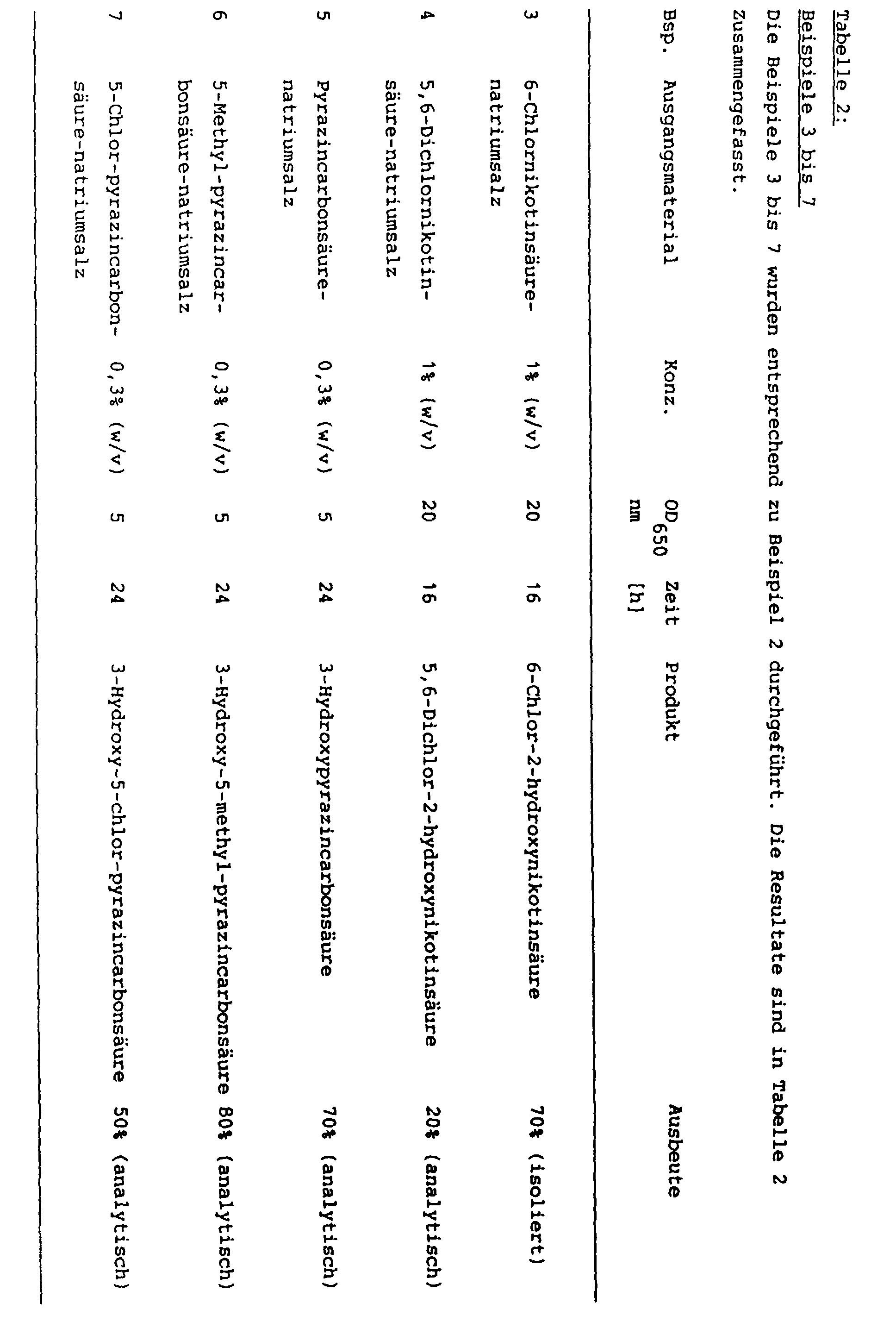

### Analytische Daten zu Beispiel 6:

### (3-Hydroxy-5-methyl-pyrazincarbonsäure)

¹H-NMR: (DMSO, 400 MHz) δ in ppm
2,4, s; 2,6, s; 3,8, s; 7,8,s.

¹³C-NMR: (DMSO, 100,5 MHz) δ in ppm
20, t; 40, m; 130, s; 134, s; 155, s; 164, s; 170, s.

### Analytische Daten zu Beispiel 7:

### (3-Hydroxy-5-chlor-pyrazincarbonsäure)

¹H-NMR: (D₂O und Dioxan, 400 MHz) δ in ppm
3,8, s; 4,7, s; 8,0, s.

¹³C-NMR: (D₂O und Dioxan, 100,5 MHz) δ in ppm
132, s; 133, s; 145, s; 149, s; 164, s; 172, s.

## Patentansprüche

1. Mikrobiologisches Verfahren zur Herstellung von Hydroxy-Stickstoff-Heterocyclus-Carbonsäuren oder deren lösliche Salze, der allgemeinen Formel 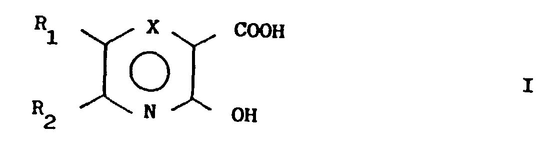 worin R₁ und R₂ gleich oder verschieden sind und ein Wasserstoffatom, ein Halogenatom oder eine C₁-C₄-Alkylgruppe und X ein Stickstoffatom oder eine CR₃-Funktion bedeuten, worin R₃ ein Wasserstoff- oder ein Halogenatom bedeutet, dadurch gekennzeichnet, dass man eine Stickstoff-Heterocyclus-Carbonsäure oder deren lösliche Salze der allgemeinen Formel 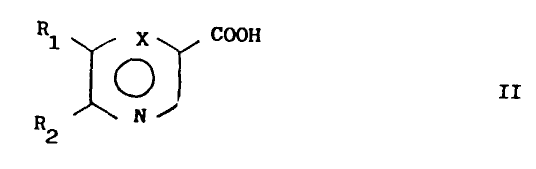 worin R₁, R₂ und X die genannte Bedeutung haben, als Substrat, mittels 6-Methylnikotinsäure-verwertenden Mikroorganismen enthaltend eine spezifische Hydroxylase, die derart selektioniert sind, daß sie 6-Methylnikotinsäure als einzige Kohlenstoff-, Stickstoff- und Energiequelle über 2-Hydroxy-6-methylnikotinsäure verwerten, in das Produkt gemäß Formel I überführt.

2. Verfahren nach Patentanspruch 1, dadurch gekennzeichnet, dass man als Substrat Stickstoff-Heterocyclus-Carbonsäuren der allgemeinen Formel 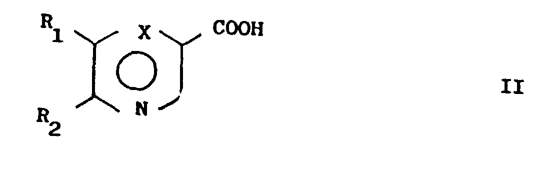 verwendet, worin R₁ und R₂ gleich oder verschieden sind und ein Wasserstoffatom, ein Chloratom, oder eine Methylgruppe und X ein Stickstoffatom oder eine CH-Funktion bedeuten.

3. Verfahren nach mindestens einem der Patentansprüche 1 und 2, dadurch gekennzeichnet, dass man die Umsetzung mit Mikroorganismen der Bezeichnung KilOl (DSM 6920) oder deren Deszendenten und Mutanten durchführt.

4. Verfahren nach mindestens einem der Patentansprüche 1 bis 3, dadurch gekennzeichnet, dass die Umsetzung unter einmaliger oder kontinuierlicher Substratzugabe erfolgt, so dass die Substratkonzentration im Kulturmedium 10 Gew.% nicht übersteigt.

5. Verfahren nach mindestens einem der Patentansprüche 1 bis 4, dadurch gekennzeichnet, dass man die Umsetzung bei einer Temperatur von 15 bis 50°C und bei einem pH von 5 bis 9 durchführt.

6. Mikroorganismen, die derart selektioniert sind, dass sie 6-Methylnikotinsäure als einzige Kohlenstoff-, Stickstoff- und Energiequelle über 2-Hydroxy-6-methylnikotinsäure verwerten.

7. Mikroorganismen nach Patentanspruch 6, mit der Bezeichnung Ki101 (DSM 6920) sowie deren Deszendenten und Mutanten.

## Claims

1. Microbiological process for the production of hydroxy nitrogen-heterocycle carboxylic acids or the soluble salts thereof of the general formula 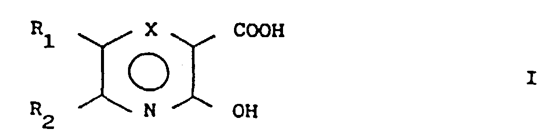 in which R₁ and R₂ are identical or different and mean a hydrogen atom, a halogen atom or a C₁-C₄ alkyl group and X means a nitrogen atom or a CR₃ function, in which R₃ means a hydrogen or halogen atom, characterised in that a nitrogen-heterocycle carboxylic acid or the soluble salts thereof of the general formula 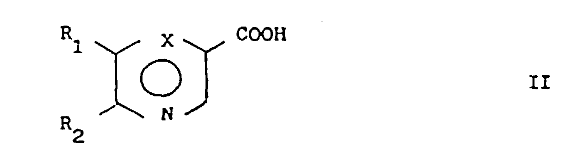 in which R₁, R₂ and X have the stated meaning, as the substrate, are converted by means of 6-methylnicotinic acid metabolising microorganisms containing a specific hydroxylase, which are selected by the fact that they metabolise 6-methylnicotinic acid via 2-hydroxy-6-methylnicotinic acid as their sole source of carbon, nitrogen and energy, to yield the product of the formula I.

2. Process according to claim 1, characterised in that nitrogen-heterocycle carboxylic acids of the general formula 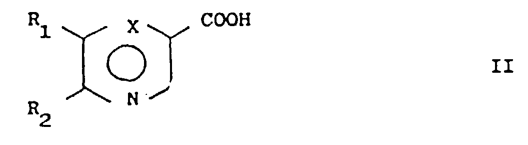 are used as the substrate, in which R₁ and R₂ are identical or different and mean a hydrogen atom, a chlorine atom, or a methyl group and X means a nitrogen atom or a CH function.

3. Process according to at least one of claims 1 and 2, characterised in that the conversion is performed with microorganisms of the designation Ki101 (DSM 6920) or the descendants and mutants thereof.

4. Process according to at least one of claims 1 to 3, characterised in that conversion proceeds with the substrate being added continuously or in a single portion in such a manner that the substrate concentration in the culture medium does not exceed 10 wt.%.

5. Process according to at least one of claims 1 to 4, characterised in that conversion is performed at a temperature of 15 to 50°C and at a pH of 5 to 9.

6. Microorganisms which are selected by the fact that they metabolise 6-methylnicotinic acid via 2-hydroxy-6-methylnicotinic acid as their sole source of carbon, nitrogen and energy.

7. Microorganisms according to claim 6, designated Ki101 (DSM 6920) and the descendants and mutants thereof.

## Revendications

1. Procédé microbiologique pour l'hydroxylation d'acides carboxyliques hétérocycles contenant de l'azote ou de leurs sels solubles, selon la formule générale 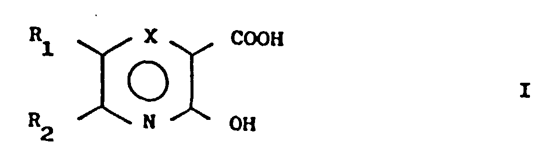 dans laquelle R₁ et R₂ sont identiques ou différents et signifient un atome hydrogène, un atome halogène ou un groupe alkyle C₁ - C₄ et X signifie un atome azote ou une fonction CR₃, R₃ signifiant un atome hydrogène ou halogène, caractérisé en ce que l'on transforme un acide carboxylique hétérocycle contenant de l'azote ou ses sels solubles de la formule générale 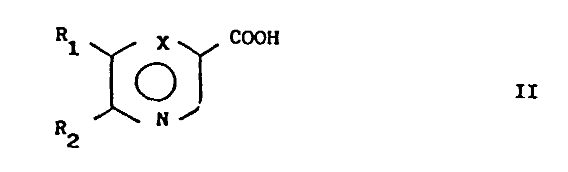 dans laquelle R₁, R₂ et X ont la signification citée, en tant que substrat, au moyen de microorganismes utilisant l'acide 6-méthylnicotinique contenant une hydroxylase spécifique, qui sont sélectionnés de telle sorte que l'acide 6-méthylnicotinique sert de seule et unique source d'énergie, d'azote et de carbone, en le produit selon la formule I.

2. Dispositif selon la revendication 1, caractérisé en ce qu'en tant que substrat, on utilise des acides carboxyliques hétérocycles contenant de l'azote de la formule générale 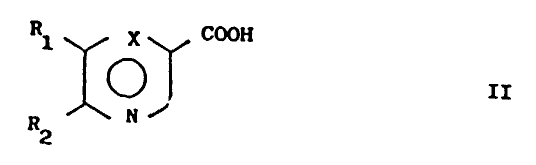 dans laquelle R₁ et R₂ sont identiques ou différents et signifient un atome hydrogène, un atome chlore, ou un groupe méthyle et X un atome azote ou une fonction CH.

3. Dispositif selon au moins l'une des revendications 1 et 2, caractérisé en ce que l'on effectue la réaction avec des microorganismes de la désignation Ki101 (DSM 6920) ou leurs descendants et mutants.

4. Dispositif selon au moins l'une des revendications 1 à 3, caractérisé en ce que la réaction s'effectue sous addition de substrat en une seule fois ou de façon continue, de sorte que la concentration de substrat dans le milieu de culture ne dépasse pas 10 % en poids.

5. Procédé selon au moins l'une des revendications 1 à 4, caractérisé en ce que l'on conduit la réaction à une température de 15 à 50°C et à un pH de 5 à 9.

6. Microorganismes qui sont sélectionnés de telle sorte qu'ils utilisent l'acide 6-méthylnicotinique en tant que seule et unique source de carbone, d'azote et d'énergie par l'intermédiaire de l'acide 2-hydroxy-6-méthylnicotinique.

7. Microorganismes selon la revendication 6, portant la désignation Ki101 (DSM 6920) ainsi que leurs descendants et mutants.
